# EUROPÄISCHE PATENTSCHRIFT

(11) **EP 1 558 262 B1**
(45) Veröffentlichungstag und Bekanntmachung des Hinweises auf die Patenterteilung: **19.12.2007**
(21) Anmeldenummer: 03809321.7
(22) Anmeldetag: 24.10.2003
(51) Int. Cl.: A61K 31/5415, A61K 9/16, A61K 9/00, A61P 11/00, A61P 29/00

(54) **WASSERLÖSLICHE MELOXICAM GRANULATE**
WATER-SOLUBLE MELOXICAM GRANULATES
GRANULES DE MELOXICAM SOLUBLES DANS L'EAU

(30) Priorität: 25.10.2002 DE 10250081
(43) Veröffentlichungstag der Anmeldung: 03.08.2005
(73) Patentinhaber: Boehringer Ingelheim Vetmedica GmbH, 55216 Ingelheim am Rhein (DE)
(72) Erfinder: FOLGER, Martin, Andreas, 55218 INGELHEIM (DE); HENKE, Stefan, 57548 Kirchen (DE); SCHMALZ, Jens, 55595 HÜFFELSHEIM (DE); KEILHOFER, Diana, Christine, 55118 MAINZ (DE); KROFF, Hans-Jürgen, 55444 SCHÖNEBERG (DE); HERZ, Nina, 55452 Windesheim (DE)
(86) Internationale Anmeldenummer: PCT/EP2003/011802
(87) Internationale Veröffentlichungsnummer: WO 2004/037264

(56) Entgegenhaltungen:
- EP-A- 0 945 134
- WO-A-01/97813

## Beschreibung

Die vorliegende Erfindung betrifft in Wasser schnelllösliche Meloxicam Granulate, enthaltend Meloxicam, einen das Meglumin-, Natrium-, Kalium- oder Ammoniumsalz des Meloxicams bildenden Salzbildners, Bindemittel, einen Zucker oder Süßstoff, einen Carrier, gegebenenfalls einen Aromastoff und gegebenenfalls weitere Hilfsstoffe, Verfahren zu ihrer Herstellung, sowie ihre Verwendung für die Behandlung von respiratorischen oder entzündlichen Erkrankungen bei Säugetieren.

### Hintergrund der Erfindung

Meloxicam (4-Hydroxy-2-methyl-N-(5-methyl-2-thiazolyl)-2H-1,2-benzothiazin-3-carboxamid-1,1-dioxid) ist ein zur Gruppe der NSAID's (non-steroidal-antiinflammatory drugs) gehörender Wirkstoff. Meloxicam sowie dessen Natrium- und Megluminsalz (N-Methyl-D-glucaminsalz) sind in EP-A-0 002 482 beschrieben. EP A-0 945 134 offenbart das pH-abhängige Löslichkeitsverhalten von Meloxicam und seinen Salzen, d.h. des Natriumsalzes, des Ammoniumsalzes und des Megluminsalzes, in wässriger Lösung. Danach ist Meloxicam ein in Wasser schwerlöslicher Wirkstoff. Die Meloxicamsalze, insbesondere das Megluminsalz, zeigen wie in Tabelle 1 aus EP 0 945 134 gezeigt eine verbesserte Löslichkeit mit steigendem pH-Wert zwischen 4 und 10.

Es ist bekannt, dass die Verabreichung von Arzneimitteln an erkrankte, insbesondere an fiebererkrankte, Tiere besonders einfach und erfolgreich über das Trinkwasser durchgeführt werden kann. Eine Verabreichung über das Futter kann ebenfalls die Zuführung des Arzneimittels an das Tier erleichtern. Aus EP 0945134 ist bekannt, dass eine einfache Verpressung von Meloxicam und Meglumin nicht möglich ist.

Es ist daher die Aufgabe der vorliegenden Erfindung ein Meloxicamgranulat zu entwickeln, das durch Einmischen in die Tränke der Tiere oder als Futteraufgabe diesen verabreicht wird.

### Beschreibung der Erfindung

Überraschenderweise wurden Meloxicam Granulate gefunden, welche nach einem Wirbelschichtverfahren auf einfache Weise hergestellt werden können und nach schnellem Auflösen in Wasser über mindestens 48 Stunden eine stabile Trinkwasserlösung bilden. Es wurde ebenfalls gefunden, dass diese Granulate als Futteraufgabe verabreicht werden können.

Gegenstand der Erfindung sind daher wasserlösliche Granulate enthaltend Meloxicam, einen das Meglumin-, Natrium-, Kalium- oder Ammoniumsalz des Meloxicams bildenden Salzbildners, Bindemittel, einen Zucker oder Süßstoff, einen Carrier, gegebenenfalls einen Aromastoff sowie gegebenenfalls weitere Hilfsstoffe.

Die erfindungsgemäßen Meloxicam Granulate zeigen mehrere Vorteile im Vergleich zu vorhandenen Darreichungsformen.

Bei kranken Tieren kann eine erhöhte Trinkwasseraufnahme bei Verabreichung einer meloxicamhaltigen Tränke festgestellt werden. Eine beliebige Verdünnung des gelösten Granulats ermöglicht eine variable genaue Dosierung des Wirkstoffs Meloxicam. Durch die gute Wasserlöslichkeit der erfindungsgemäßen Meloxicam Granulate ist eine sehr schnell eintretenden Wirkung im Körper des erkrankten Tieres gegeben. Der gute Geschmack der Meloxicam Granulate ermöglicht auch die Verabreichung als Futteraufgabe.

Außerdem weisen die erfindungsgemäßen Granulate eine sehr gute Fließfähigkeit, eine Gleichförmigkeit des Meloxicamgehalts, nahezu Staubfreiheit und eine enge Partikelgrößenverteilung von 125 *µ*m bis 500 *µ*m auf.

Die vollständige Löslichkeit des Granulats in Wasser gewährleistet die optische Kontrolle eines vollständig gelösten Wirkstoffs, welcher nur in dieser Form der therapeutischen Anwendung bei der Trinkwasserapplikation zur Verfügung steht.

In einer bevorzugten Ausführungsform der Erfindung ist der Salzbildner Meglumin.

In einer ebenfalls bevorzugten Ausführungsform der Erfindung kann das Bindemittel ausgewählt sein aus der Gruppe bestehend aus Hydroxypropyl-methylcellulose, Polyvinylpyrrolidone, Gelatine, Stärke und Polyethylenglykolether, vorzugsweise Hydroxypropyl-methylcellulose, Polyvinylpyrrolidone und Polyethylenglykolether, besonders bevorzugt Hydroxypropyl-methylcellulose und Polyvinylpyrrolidone.

In einer weiteren bevorzugten Ausführungsform der Erfindung kann der Zucker oder Süßstoff ausgewählt sein aus der Gruppe bestehend aus Natrium Saccharin, Aspartam und Sunett®, vorzugsweise Natrium Saccharin oder Aspartam.

Besonders bevorzugt sind erfindungsgemäße Meloxicam Granulate, in welchen der Aromastoff ausgewählt ist aus der Gruppe bestehend aus Vanille, Honigaroma, Apfelaroma, und Contramarum, vorzugsweise Honigaroma und Apfelaroma.

Weiterhin besonders bevorzugt sind Meloxicam Granulate, worin der Carrier ausgewählt ist aus der Gruppe bestehend aus Lactose, Glucose, Mannitol, Xylitol, Saccharose und Sorbitol vorzugsweise Glucose, Lactose oder Sorbitol, besonders bevorzugt Glucose oder Lactose, insbesondere bevorzugt Glucose.

Insbesondere bevorzugt sind Meloxicam Granulate, worin der Anteil an Meloxicam zwischen 0,05 % und 4 % beträgt, vorzugsweise zwischen 0,1 bis 2 %, bevorzugt zwischen 0,3 % und 1,5 %, besonders bevorzugt zwischen 0,4 % und 1 %, insbesondere bevorzugt 0,6 %. Weiterhin insbesondere bevorzugt sind Meloxicam Granulate, welche Meglumin und

Meloxicam in einem molaren Verhältnis von 9:8 bis 12:8, bevorzugt 10:8 enthalten.

Ein weiterer Gegenstand der Erfindung sind Verfahren zur Herstellung der erfindungsgemäßen Meloxicam Granulate, worin die aufeinanderfolgenden Schritte a) bis c) durchgeführt werden:
a) Herstellung einer wässrigen Granulierflüssigkeit enthaltend Bindemittel, gegebenenfalls einen Zucker oder Süßstoff, Meloxicam, Meglumin und/oder ein Aromastoff.
b) Aufsprühen der Granulierflüssigkeit auf einen Carrier in einem Topspray-Wirbelschichtverfahren bei einem konstant temperierten Zuluftstrom von 50 bis 80°C, vorzugsweise 65 °C.
c) Anschließen eines Coatingprozesses mit einer wässrigen Granulierflüssigkeit im Topspray-Wirbelschichtverfahren enthaltend ein Bindemittel, einen Zucker oder Süßstoff und/oder ein Aroma.

Bevorzugt wird ein erfindungsgemäßen Verfahren worin die Granulierflüssigkeit durch Rühren und Erhitzen der Komponenten auf 70 bis 100 °C, vorzugsweise etwa 90 °C hergestellt wird.

Ein besonderes Merkmal der erfindungsgemäßen Meloxicam Granulate ist, dass sie bei Raumtemperatur in Originalverpackung eine Langzeitstabilität von 24 Monaten oder mehr aufweisen.

Ein überaus bevorzugtes Meloxicamgranulat enthält Meloxicam, Meglumin, Hydroxypropylmethylcellulose, Povidone und Glucose Monohydrat.

Ein weiterer Gegenstand der vorliegenden Erfindung ist die Verwendung eines Meloxicamgranulats zur Herstellung eines Arzneimittels zur Behandlung von Schmerz, Entzündungen, Fieber, akuter Mastitis, Diarrhoe, Lahmheiten, Beeinträchtigungen des Bewegungsapparates und respiratorischen Erkrankungen bei Tieren, vorzugsweise von akuter Mastitis, Diarrhoe, Lahmheiten, Beeinträchtigungen des Bewegungsapparates und respiratorischen Erkrankungen, bevorzugt von akuter Mastitis, Diarrhoe, Lahmheiten, Beeinträchtigungen des Bewegungsapparates und respiratorischen Erkrankungen, insbesondere bevorzugt von Beeinträchtigungen des Bewegungsapparates oder respiratorischen Erkrankungen. Die Behandlung kann in Verbindung mit einer antibiotischen Therapie erfolgen.

Die erfindungsgemäße Formulierung eignet sich zur Behandlung von Tieren, bevorzugt von Säugetieren, insbesondere Haustieren oder Nutztieren, beispielsweise Schweine, Pferde, Rinder, Hunde oder Katzen, bevorzugt Schweine oder Pferde.

Bevorzugt ist die Verwendung des erfindungsgemäßen Meloxicamgranulats, welche einem Dosierungsbereich von 0,2 bis 1,0 mg Wirkstoff/kg Körpergewicht, vorzugsweise 0,4 bis 0,8 mg/kg Köpergewicht, bevorzugt 0,5 bis 0,7 mg/kg Köpergewicht, insbesondere bevorzugt 0,6 mg/kg Köpergewicht entspricht.

Ebenfalls bevorzugt ist die Verwendung des erfindungsgemäßen Meloxicamgranulats zur Herstellung eines Arzneimittels, welches sowohl über die Tränke als auch als Futteraufgabe verabreicht werden kann.

Die erfindungsgemäße Formulierung kann als Meloxicamsalz das Meglumin-, Natrium-, Kalium oder Ammoniumsalz, bevorzugt das Meloxicam Megluminsalz enthalten.

Der Anteil an Meglumin liegt zwischen 0,035 und 2,8 %, vorzugsweise 0,07 bis 1,4 % bevorzugt 0,21-1,05 %, besonders bevorzugt 0,28-0,7 % mg/g, insbesondere 0,42 % im Meloxicam Granulat. Die möglichen Natrium-, Kalium- und Ammoniumkonzentrationen berechnen sich entsprechend.

Die Konzentration der Bindemittel kann im Bereich von 20-80 mg/g, vorzugsweise 30-70 mg/g, bevorzugt 40-60 mg/g, besonders bevorzugt 50 mg/g Granulat liegen.

Die Konzentration des Zuckers kann im Bereich von 50-150 mg/g, vorzugsweise 75-125 mg/g, besonders bevorzugt etwa 100 mg/g Granulat liegen.

Die Konzentration des Süßstoffs kann im Bereich von 1-10 mg/g, vorzugsweise 2-5 mg/g, besonders bevorzugt etwa 3 mg/g Granulat liegen.

Die Konzentration des Carriers kann im Bereich von 800-985 mg/g, vorzugsweise 900-960 mg/g, besonders bevorzugt etwa 930 mg/g Granulat liegen.

Die Konzentration des Aromastoffs kann im Bereich von 0,1-10 mg/g, vorzugsweise 0,2-1,0 mg/g, besonders bevorzugt etwa 0,5 mg/g Granulat liegen.

Als Verpackungsmaterial der erfindungsgemäßen Formulierung sind verschiedene handelsübliche Materialien für Granulate geeignet. Dazu gehören z.B. Kunststoffbehälter, beispielsweise aus HDPE (Hochdruckpolyethylen), Aluminiumbeutel oder Papierbeutel mit Aluminiumbeschichtung.

Die Herstellung des Meloxicam-Granulats erfolgt nach dem Top Spray Wirbelschicht Verfahren. Dazu wird zunächst eine wässrige Granulierflüssigkeits-Lösung aus 50 bis 70 g/kg Bindemittel, beispielsweise PVP 25000, Hydroxypropylmethyl-Cellulose oder Macrogol 6000, vorzugsweise Hydroxypropylmethyl-Cellulose und/ oder etwa 1 bis 5 g/kg Süßstoffen, beispielsweise Sunett® oder Na Saccharin, vorzugsweise Sunett®, und/oder 0,5 bis 2,5 g Aromen, beispielsweise Vanille, Honig, Aroma 203180 oder Contramarum, vorzugsweise Honig, 10 bis 15 g Meloxicam (peg milled) und 7 bis 11 g Meglumin unter Rühren durch Erhitzen auf 70 bis 100 °C hergestellt.

Die Granulierflüssigkeit wird anschließend auf einen Carrier beispielsweise Lactose, Glucose oder Sorbitol, vorzugsweise Glucose, im Gegenstromverfahren (Top Spray) aufgesprüht. Dies geschieht beispielsweise über eine Zweistoffdüse unter Zerstäubung mit konstantem Luftdruck bei 50 bis 80 °C , vorzugsweise bei 65 °C.

Danach kann ein Coating Prozess mit einer zweiten wässrigen Granulierflüssigkeit erfolgen. Zur Herstellung einer Anwendungslösung sollte eine Stammlösung in Wasser vollständig gelöst werden. Anschließend kann die Stammlösung durch Zumischen von Wasser auf die gewünschte Anwendungskonzentration eingestellt werden.

Zur Erhöhung der Anwendungssicherheit können die Granulate mit wasserlöslichen Farbmarkierungen versehen werden.

Die erfindungsgemäßen Meloxicam Granulate sollen durch das nachfolgende Beispiel erläutert werden. Dem Fachmann ist bewußt, daß dieses Beispiel nur zur Veranschaulichung dient.

### Beispiel 1

| 0,6%iges Meloxicamgranulat | |
|---|---|
| Rezeptur: | |
| | g/100 g |
| Meloxicam | 0,6 |
| Meglumin | 0,42 |
| Hydroxypropylmethylcellulose | 3,00 |
| Povidone | 2,00 |
| Glucose monohydrat | 93,98 |

### Beispiel 2

| 1,2%iges Meloxicamgranulat | |
|---|---|
| Meloxicam | 1,2 |
| Meglumin | 0,84 |
| Hydroxypropylmethylcellulose | 3,00 |
| Kolidon 25 | 2,0 |
| Glucose Monohydrat | 92,96 |

### Beispiel 3

| 0,6%iges Meloxicamgranulat | |
|---|---|
| Meloxicam | 0,6 |
| Meglumin | 0,42 |
| Pharmacoat 606 | 4,0 |
| Makrogol 6000 | 1,0 |
| Acesulfam K | 0,3 |
| Lactose | 93,68 |

### Beispiel 4

| 0,6%iges Meloxicamgranulat | |
|---|---|
| Meloxicam | 0,6 |
| Meglumin | 0,42 |
| Pharmacoat 606 | 4,75 |
| Makrogol 6000 | 0,25 |
| Acesulfam K | 0,3 |
| Vanille Aromaflüssig | 0,05 |
| Lactose | 93,63 |

Hellgelbe frei fließende Meloxicam-Granulate entsprechend Beispiel 1 bis 4 können wie folgt hergestellt werden:

Das Granulat wurde 3 Monate bei 25 °C und einer relativen Luftfeuchtigkeit von 60 % gelagert. Es wurden hinsichtlich des Wirkstoffsgehalts, des Wassergehalts (nach Karl-Fischer), des visuellen Auflösungsverhaltens, des pH-Werts in entmineralisiertem Wasser und der visuellen Benetzbarkeit keine signifikanten Veränderungen beobachtet.

Zur Bestimmung des visuellen Auflösungsverhaltens wurden 5 g des Granulats in 100 ml entmineralisiertem Wasser bei Raumtemperatur gelöst. Nach etwa 1 min lag eine klare gelbliche Lösung vor.

## Patentansprüche

1. Wasserlösliche Granulate enthaltend Meloxicam, Bindemittel, einen Zucker oder Süßstoff, einen Carrier, einen das Meglumin-, Natrium-, Kalium- oder Ammoniumsalz des Meloxicams bildenden Salzbildners, gegebenenfalls einen Aromastoff sowie gegebenenfalls weitere Hilfsstoffe.

2. Meloxicam Granulate nach Anspruch 1, **dadurch gekennzeichnet dass** der Salzbildner Meglumin ist.

3. Meloxicam Granulate nach Anspruch 1 oder 2, **dadurch gekennzeichnet dass** das Bindemittel ausgewählt ist aus der Gruppe bestehend aus Hydroxypropylmethylcellulose, Polyvinylpyrrolidone, Gelatine, Stärke und Polyethylenglykolether.

4. Meloxicam Granulate nach einem der Ansprüche 1 bis 3 , **dadurch gekennzeichnet dass** der Zucker oder Süßstoff ausgewählt sind aus der Gruppe bestehend Natrium Saccharin, Aspartam und Sunett®.

5. Meloxicam Granulate nach einem der Ansprüche 1 bis 4 , **dadurch gekennzeichnet dass** der Aromastoff ausgewählt ist aus der Gruppe bestehend aus Vanille, Honigaroma, Apfelaroma, und Contramarum.

6. Meloxicam Granulate nach einem der Ansprüche 1 bis 5 , **dadurch gekennzeichnet dass** der Carrier ausgewählt ist aus der Gruppe bestehend Lactose, Glucose, Mannitol, Xylitol, Saccharose und Sorbitol.

7. Meloxicam Granulate nach einem der Ansprüche 1 bis 6 , **dadurch gekennzeichnet dass** der Anteil an Meloxicam zwischen 0,05 % und 4 % beträgt.

8. Granulate nach einem der Ansprüche 1 bis 7, **dadurch gekennzeichnet, daß** sie Meglumin und Meloxicam in einem molaren Verhältnis von 9:8 bis 12:8 enthalten.

9. Granulate nach einem der Ansprüche 1 bis 8, **dadurch gekennzeichnet, daß** sie Meglumin und Meloxicam in einem molaren Verhältnis von 10:8 enthalten.

10. Verfahren zur Herstellung der Granulate gemäß einem der Ansprüche 1 bis 9, **dadurch gekennzeichnet, dass** die aufeinanderfolgenden Schritte a) bis c) durchgeführt werden:
a) Herstellung einer wässrigen Granulierflüssigkeit enthaltend ein Bindemittel, einen Zucker oder Süßstoff, Meloxicam, Meglumin und/oder ein Aromastoff.
b) Aufsprühen der Granulierflüssigkeit auf einen Carrier in einem Topspray-Wirbelschichtverfahren bei einem konstant temperierten Zuluftstrom von 50 bis 80 °C.
c) Anschließen eines Coatingprozesses mit einer wässrigen Granulierflüssigkeit im Topspray-Wirbelschichtverfahren enthaltend ein Bindemittel, einen Zucker oder Süßstoff und/oder ein Aroma.

11. Verfahren nach Anspruch 10 **dadurch gekennzeichnet, dass** die Granulierflüssigkeit durch Rühren und Erhitzen der Komponenten auf 70 bis 100 °C hergestellt wird.

12. Granulat nach einem der Ansprüche 1- 9, **dadurch gekennzeichnet, daß** sie bei Raumtemperatur in Originalverpackung eine Langzeitstabilität von 24 Monaten oder mehr aufweist.

13. Granulat nach einem der Ansprüche 1 - 9 oder 12, enthaltend Meloxicam, Meglumin, Hydroxypropylmethylcellulose, Povidone und Glucose Monohydrat.

14. Verwendung eines Meloxicamgranulats gemäß einem der Ansprüche 1 bis 9 oder 12 zur Herstellung eines Arzneimittels zur Behandlung von Schmerz, Entzündungen, Fieber und respiratorischen Erkrankungen bei Tieren.

15. Verwendung eines Meloxicamgranulats nach Anspruch 13, welches einem Dosierungsbereich von 0,2 bis 1,0 mg Wirkstoff/kg Körpergewicht entspricht.

16. Verwendung eines Meloxicamgranulats nach Anspruch 12 oder 13 zur Herstellung eines Arzneimittels, welches sowohl über die Tränke als auch als Futteraufgabe verabreicht werden kann.

## Claims

1. Water soluble granules containing meloxicam, binders, a sugar or sweetener, a carrier, a salt forming agent which forms the meglumin, sodium, potassium or ammonium salt of meloxicam, optionally a flavouring and optionally other excipients.

2. Meloxicam granules according to claim 1, **characterised in that** the salt forming agent is meglumin.

3. Meloxicam granules according to claim 1 or 2, **characterised in that** the binder is selected from among hydroxypropylmethylcellulose, polyvinylpyrrolidone, gelatine, starch and polyethyleneglycolether.

4. Meloxicam granules according to one of claims 1 to 3 , **characterised in that** the sugar or sweetener is selected from among sodium saccharine, aspartame and Sunett®.

5. Meloxicam granules according to one of claims 1 to 4 , **characterised in that** the flavouring is selected from among vanilla, honey flavouring, apple flavouring and contramarum.

6. Meloxicam granules according to one of claims 1 to 5 , **characterised in that** the carrier is selected from among lactose, glucose, mannitol, xylitol, sucrose and sorbitol.

7. Meloxicam granules according to one of claims 1 to 6 , **characterised in that** the proportion of meloxicam is between 0.05 % and 4 %.

8. Granules according to one of claims 1 to 7, **characterised in that** they contain meglumin and meloxicam in a molar ratio of 9:8 to 12:8.

9. Granules according to one of claims 1 to 8, **characterised in that** they contain meglumin and meloxicam in a molar ratio of 10:8.

10. Process for preparing the granules according to one of claims 1 to 9, **characterised in that** the steps a) to c) are carried out successively:
a) Preparing an aqueous granulating liquid containing a binder, a sugar or sweetener, meloxicam, meglumin and/or a flavouring.
b) Spraying the granulating liquid on to a carrier in a topspray fluidised bed method with a supply of air at a constant temperature of 50 to 80 °C.
c) A subsequent coating process with an aqueous granulating liquid by the topspray fluidised bed method containing a binder, a sugar or sweetener and/or a flavouring.

11. Process according to claim 10, **characterised in that** the granulating liquid is prepared by stirring and heating the components to 70 to 100°C.

12. Granules according to one of claims 1- 9, **characterised in that** they have a long term stability of 24 months or longer when stored at ambient temperature in their original packaging.

13. Granules according to one of claims 1 - 9 or 12, containing meloxicam, meglumin, hydroxypropylmethylcellulose, povidone and glucose monohydrate.

14. Use of meloxicam granules according to one of claims 1 to 9 or 12 for preparing a pharmaceutical composition for the treatment of pain, inflammation, fever and respiratory complaints in animals.

15. Use of meloxicam granules according to claim 13, which corresponds to a dosage range of from 0.2 to 1.0 mg of active substance per kg of bodyweight.

16. Use of meloxicam granules according to claim 12 or 13 for preparing a pharmaceutical composition which can be administered both in drink and feed supplements.

## Revendications

1. Granulés solubles dans l'eau contenant du méloxicam, un liant, un sucre ou édulcorant, un vecteur, un agent salifiant formant le sel de méglumine, de sodium, de potassium ou d'ammonium du méloxicam, éventuellement un arôme et éventuellement d'autres adjuvants.

2. Granulés de méloxicam selon la revendication 1 **caractérisés en ce que** l'agent salifiant est la méglumine.

3. Granulés de méloxicam selon la revendication 1 ou 2 **caractérisés en ce que** le liant est choisi dans le groupe consistant en l'hydroxypropylméthylcellulose, la polyvinylpyrrolidone, la gélatine, l'amidon et le polyéthylèneglycoléther.

4. Granulés de méloxicam selon l'une des revendications 1 à 3 **caractérisés en ce que** le sucre ou édulcorant est choisi dans le groupe consistant en la saccharine sodique, l'aspartam et Sunett®.

5. Granulés de méloxicam selon l'une des revendications 1 à 4 **caractérisés en ce que** l'arôme est choisi dans le groupe consistant en la vanille, l'arôme de miel, l'arôme de pomme et contramarum.

6. Granulés de méloxicam selon l'une des revendications 1 à 5 **caractérisés en ce que** le vecteur est choisi dans le groupe consistant en le lactose, le glucose, le mannitol, le xylitol, le saccharose et le sorbitol.

7. Granulés de méloxicam selon l'une des revendications 1 à 6 **caractérisés en ce que** la proportion de méloxicam est de 0,05 % à 4 %.

8. Granulés selon l'une des revendications 1 à 7 **caractérisés en ce qu'**ils contiennent de la méglumine et du méloxicam dans un rapport molaire de 9 : 8 à 12 : 8.

9. Granulés selon l'une des revendications 1 à 8 **caractérisés en ce qu'**ils contiennent de la méglumine et du méloxicam dans un rapport molaire de 10 : 8.

10. Procédé de production des granulés selon l'une des revendications 1 à 9 **caractérisé en ce que** les étapes a) à c) successives sont accomplies :
a) production d'un liquide de granulation aqueux contenant un liant, un sucre ou édulcorant, du méloxicam, de la méglumine et/ou un arôme,
b) pulvérisation du liquide de granulation sur un vecteur dans un procédé en lit fluidisé topspray avec un courant d'air entrant maintenu à température constante de 50 à 80°C,
c) puis un procédé d'enrobage avec un liquide de granulation aqueux dans le procédé en lit fluidisé topspray contenant un liant, un sucre ou édulcorant et/ou un arôme.

11. Procédé selon la revendication 10 **caractérisé en ce que** le liquide de granulation est produit par agitation et chauffage des composants à 70 à 100°C.

12. Granulés selon l'une des revendications 1-9 **caractérisés en ce qu'**ils présentent à la température ambiante dans l'emballage d'origine une stabilité à long terme de 24 mois ou plus.

13. Granulés selon l'une des revendications 1-9 ou 12 contenant du méloxicam, de la méglumine, de l'hydroxypropylméthylcellulose, de la povidone et du glucose monohydraté.

14. Utilisation de granulés de méloxicam selon l'une des revendications 1 à 9 ou 12 pour la production d'un médicament pour le traitement de la douleur, des inflammations, de la fièvre et des maladies respiratoires chez les animaux.

15. Utilisation de granulés de méloxicam selon la revendication 13 qui correspondent à un domaine posologique de 0,2 à 1,0 mg de principe actif/kg de poids corporel.

16. Utilisation de granulés de méloxicam selon la revendication 12 ou 13 pour la production d'un médicament qui peut être administré aussi bien par le biais de l'abreuvoir que par le biais de l'apport de nourriture.
